Europäisches Patentamt

European Patent Office

Office européen des brevets

(11) Veröffentlichungsnummer: **0 642 806 A2**

# (12) EUROPÄISCHE PATENTANMELDUNG

(21) Anmeldenummer: **94110949.8**

(22) Anmeldetag: **14.07.94**

(51) Int. Cl.⁶: **A61N 1/16, A61N 2/00**

(30) Priorität: **06.09.93 DE 4329884**

(43) Veröffentlichungstag der Anmeldung:
**15.03.95 Patentblatt 95/11**

(84) Benannte Vertragsstaaten:
**CH FR GB IT LI**

(71) Anmelder: **Ruhenstroth-Bauer, Gerhard, Prof. Dr.**
**Spietzelbergerstrasse 11**
**D-82166 Gräfeling (DE)**

(72) Erfinder: **Ruhenstroth-Bauer, Gerhard, Prof. Dr.**
**Spietzelbergerstrasse 11**
**D-82166 Gräfeling (DE)**

(74) Vertreter: **Dreiss, Hosenthien, Fuhlendorf & Partner**
**Gerokstrasse 6**
**D-70188 Stuttgart (DE)**

(54) Verfahren und Vorrichtung zur Beeinflussung derjenigen Eigenschaften biologischen Materials, die mit dem Auftreten bestimmter atmosphärischer Impulsstrahlung (Atmosperhics) korrelieren.

㉗ Beschrieben wird ein Verfahren zur Beeinflussung biologischen Materials. Beeinflußt werden sollen solche Eigenschaften, die mit dem Auftreten natürlicher Impulsstrahlung (Atmospherics) korrelieren, nämlich die Poliferationsrate von C6-Glioma-Zellen sowie die Überlebenszeit von Yoshida-Ascites-Hepatom-Ratten. Dazu werden Signale gespeichert, die den zeitlichen Verlauf des Magnetfeldes bestimmter natürlicher Impulsstrahlung darstellen, die anhand ausgeprägter Frequenzanteile, z.B. 10 kHz identifiziert werden. Aus diesen Signalen werden als artifizielle Impulsstrahlung entsprechende Magnetfeldverläufe reproduziert. Die Reproduktion erfolgt z.B. in einer Helmholtz-Spule. Das biologische Material wird diesem Magnetfeld ausgesetzt.

EP 0 642 806 A2

Es ist bekannt, daß bestimmte elektromagnetische Schwingungsphänomene, die im Zusammenhang mit dem Wettergeschehen in der Atmosphäre auftreten, mit Veränderungen biologischer und pathologischer Parameter korrelieren.

Eine Charakterisierung der betreffenden Impulse (Very-Low-Frequency Atmospherics; im folgenden: Impulsstrahlung), insbesondere nach Frequenz und Wellenform, erfolgte u. a. durch H. Baumer und J. Eichmeier in: Eine Anlage zur Registrierung der Atmospherics bei 10 und 27 kHz, **Archiv für Meteorologie, Geophysik und Bioklimatologie, Ser. A, 29, 143 bis 155 (1980)**, sowie durch W. Sönning, H. Baumer und J. Eichmeier in: Die Atmospherics-Aktivität bei 10 und 27 kHz als Indikator für die Dynamik der troposphärischen Wettervorgänge, **Archives For Meteorology, Geophysics, and Bioclimatology, Ser. B, 29, 299 -312 (1981)**. Bei der Impulsstrahlung handelt es sich um kurzzeitige Impulse in Form gedämpfter Schwingungen. Ihre Dauer beträgt jeweils ca. 5 bis 6 Halbwellen. Es handelt sich um ein Gemisch verschiedener Frequenzen; die Impulsstrahlung ist von Baumer anhand ihres jeweils überwiegenden Frequenzanteil von z. B. 6, 8, 10, 12 und 28 kHz meßtechnisch bestimmt und beschrieben worden. Sie wird daher im folgenden als "nach Baumer" = nB oder "according to Baumer" = atB bezeichnet.

Die Häufigkeit des Auftretens bestimmter Impulsstrahlung wurde bereits mit verschiedenen biologischen Parametern in Zusammenhang gebracht. So ist bei H. Baumer, Die Meteotropie eines Dichromat-Gelatinesystems, **Technischer Informationsdienst des Bundesverbandes Druck e. V. II/1982, S. 1 bis 17** beschrieben, daß die Differenz der Impulsraten im 10-kHz-und im 28-kHz-Bereich deutlich mit dem Diffusionsverhalten von Gelatine korreliert, das wiederum den Formherstellungsprozeß im Tiefdruckverfahren beeinflußt; vgl. dazu ferner H. Baumer und J. Eichmeier, Relationship between the Pulse Rate of Impulsstrahlung and the Diffusion Time of Ions in Gelatine Films, **Int. J. Biometeor. 1980, Vol. 24, no. 3, pp. 271 - 276**, sowie H. Baumer und J. Eichmeier, The Biophysically Active Wave Forms of Impulsstrahlung Incident on Gelatine Films,
**Int. J. Biometeor. 1982, Vol. 26, pp. 85-90.**

Die Korrelation von Impulsstrahlung mit bestimmten Krankheiten, wie z. B. Epilepsie und Herzinfarkt, bei denen man schon früher einen Zusammenhang mit dem Wettergeschehen annahm, ist in der **EP 0 120 991 A2 (= US-PS 4,631,957)** beschrieben; vgl. dazu auch G. Ruhenstroth-Bauer, H. Baumer, u. a., Epilepsy and Weather: A Significant Correlation Between the Onset of Epileptic Seizures and Specific Impulsstrahlung - A Pilot Study, **Int. J. Biometeor., 1984, Vol. 28, no. 4, pp. 333 - 340** und G. Ruhenstroth-Bauer, H. Baumer, u. a., Myocardial Infarction and the Weather: A Significant Positive Correlation Between the Onset of Heart Infarct and 28 kHz Impulsstrahlung - A Pilot Study, **Clin. Cardiol., 8, p. 149 - 151 (1985)**.

Man hat ferner auch eine Korrelation zwischen der 8- und 10-kHz Impulsstrahlung und Entzündungsvorgängen bei Ratten festgestellt; siehe G. Ruhenstroth-Bauer, O. Rösing, and H. Baumer, **Naturwissenschaften 73, S. 625 (1986)**.

Ferner hat man auch bedeutende Korrelationen zwischen natürlichen atmosphärischen Spektren und der in vitro Inkorporation von ($^3$H)-Thymidin in die nukleare DNA von C6-Glioma-Zellen festgestellt; vgl. Vogl, G. Hoffmann, B. Stöpfel, H. Baumer, O. Kemski u. G. Ruhenstroth-Bauer, Significant correlations between atmospheric spectra according to Baumer and the in vitro incorporation of ($^3$H)-thymidine into the nuclear DNA of C6-glioma-cells, **FEBS Letters, vol. 288, no. 1, 2, pp. 244 - 246 (1991)**.

Eine Zusammenfassung der derzeit bekannten Zusammenhänge der Impulsstrahlung und biologischen und pathologischen Parametern findet sich bei G. Hoffmann, S. Vogl, H. Baumer, O. Kemski, G. Ruhenstroth-Bauer, Significant correlations between certain spectra of atmospherics and different biological and pathological parameters, in: **Int. J. Biometeorol. (1991) 34:pp. 247 -250**.

Eine **kausale** Beziehung zwischen dem Auftreten dieser Impulsstrahlung und den beschriebenen biologischen und pathologischen Parametern konnte jedoch bisher noch nicht nachgewiesen werden.

Aufgabe der Erfindung ist es, ein Verfahren und eine Vorrichtung anzugeben, mit dem bzw. mit der diejenigen Eigenschaften biologischen Materials, die mit dem Auftreten der Impulsstrahlung korrelieren, direkt beeinflußt werden können.

Erfindungsgemäß wird diese Aufgabe durch ein Verfahren und eine Vorrichtung, wie sie in den Patentansprüchen gekennzeichnet sind, gelöst.

Die Erfindung wird im folgenden anhand der beigefügten Zeichnungen näher beschrieben. Es stellen dar:

Fig. 1(a)      den zeitlichen Verlauf der magnetischen Feldstärke in A/m.$10^{-3}$ einer typischen VLF-Impulsstrahlung in Abhängigkeit von der Zeit;

Fig. 1(b)      das Frequenzspektrum der in Fig. 1(a) gezeigten Impulsstrahlung zwischen 0 und 100 kHz;

Fig. 2(a)      ein Ausführungsbeispiel einer Anordnung zur Aufnahme und Speicherung der Impulsstrahlung;

Fig. 2(b)      ein Ausführungsbeispiel einer Anordnung zur Erzeugung artifizieller Impulsstrahlung und zur Bestrahlung von C6-Glioma-Zellen;

Fig. 3 die Darstellung von Meßergebnissen bei der Beeinflussung der Proliferationsrate von C6-Glioma-Zellen durch Bestrahlung mit artifizieller Impulsstrahlung mit einem überwiegendem Frequenzanteil von 10 kHz(nB);

Fig. 4(a) die Überlebenszeit von Yoshida-Tumor-Ratten nach/ohne Bestrahlung mit einer 10 kHz artifiziellen Impulsstrahlung bei einer maximalen Feldstärke von 25 mA/m;

Fig. 4(b) wie Fig. 4(a), jedoch bei einer maximalen Feldstärke von 100 mA/m;

Fig. 5 die Verschiebung des Elektroenzephalogramms (EEG) bei Testpersonen bei Beeinflussung durch artifizielle Impulsstrahlung.

Die Beeinflussung biologischen Materials durch artifizielle Impulsstrahlung wird im folgenden für drei Fälle beschrieben, nämlich für (1) die Proliferationsrate von C6-Glioma-Zellen, (2) die Überlebenszeit von Yoshida-Ascites-Hepatom-Ratten und (3) die Verschiebung des EEGs von menschlichen Probanden.

## ALLGEMEINES

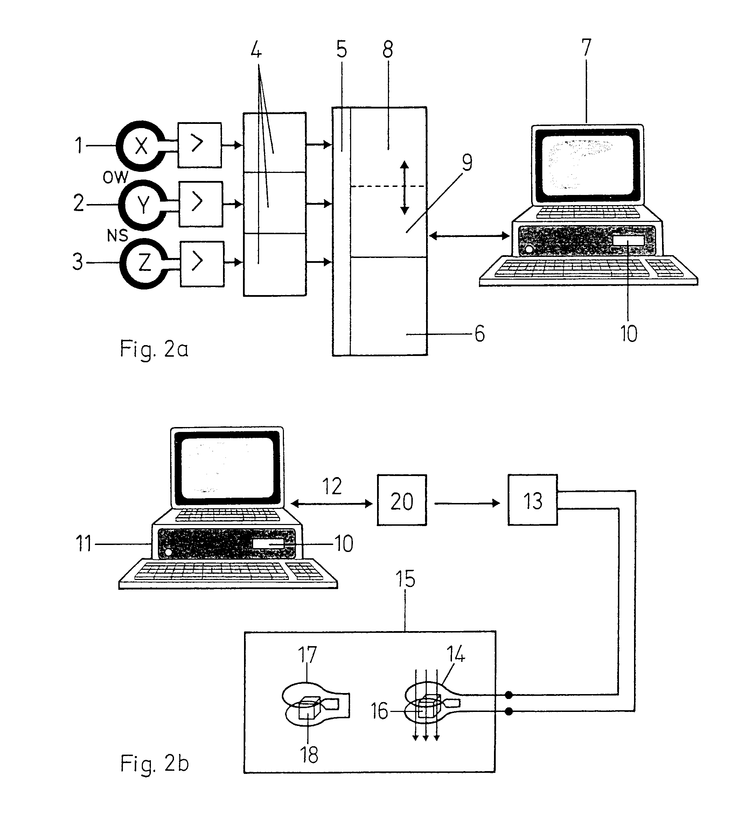

Zu Zeitpunkten, an denen eine natürliche Impulsstrahlung mit besonders ausgeprägtem Frequenzanteil von 10 kHz festgestellt wurden, wurde diese Impulsstrahlung mit einem dafür besonders ausgelegten breitbandigen Meßsystem nach Fig. 2(a) aufgenommen.

Diese Aufnahmevorrichtung ist in der Literatur von H. L. König, R. Kulzer und H.-D. Betz in: "Aufbau einer Meßstation zur breitbandigen Untersuchung von VLF-Impulsstrahlung", **Kleinheubacher Berichte, Band 35, (1992), S. 387 bis 394** und in der **P 41 33 209.1 A1** beschrieben. Dabei wird der magnetische Feldvektor mit einem System aus drei orthogonal zueinander angeordneten Antennen 1, 2, 3 registriert, die mit den Komponenten X, Y, Z und der Kompassausrichtung (OW = Ost-West, NS = Nord-Süd, Z = vertikal) beschriftet sind. Um spektrale Überlappungen bei der nachfolgenden zeitdiskreten Signalverarbeitung zu vermeiden, wird jedes der Signale in einem zugeordneten Filter 4 bandbegrenzt. Die 3 db-Punkte der Filter liegen bei 1 kHz und 100 kHz. Von dort gelangen die Signale an einen A/D-Wandler 5. Der A/D-Wandler wird über eine vom Rechner 7 aus programmierbare Triggerlogik 8 gesteuert. Hat die Triggerlogik den Einfall einer Impulsstrahlung erkannt, so wird diese, d.h. genauer: die zugehörigen digitalisierten Meßwerte auf den Rechner 7 übertragen, dort weiter bearbeitet und dann im Rechner in einem weiteren Speicher, z.B. einer Diskette oder einer Wechselfestplatte gespeichert. Die Bearbeitung im Rechner umfaßt u.a. die rechnerische Ermittlung des Absolutbetrages des magnetischen Feldvektors aus den gemessenen Komponenten. Dieser Speicher ist in Fig. 2a mit der Bezugsziffer 10 angedeutet. Die Triggerlogik 8, der Speicher 6 und ein Timer 9 befinden sich üblicherweise auf einer Platine und sind daher als Einheit dargestellt. Die Erkennung der Impulsstrahlung durch die Triggerlogik basiert auf der Überschreitung bestimmter Schwellwerte und auf der Charakterisierung von Frequenzanteilen und Schwingungsformen nach Baumer (s.oben), wenngleich die vorliegende Erfindung damit gerade weitere elektromagnetische Einflußgrößen erfaßt, nicht anders als durch eben diese Reproduktion.

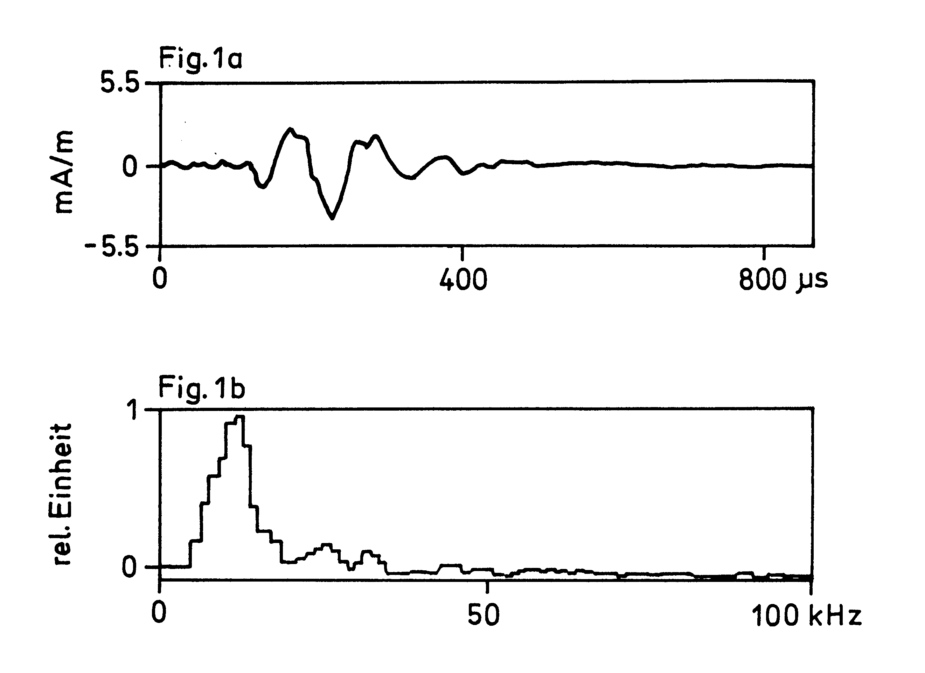

Fig. 1(a) zeigt den zeitlichen Verlauf einer so gewonnenen und abgespeicherten Impulsstrahlung. Für einen Zeitraum von 820 Mikrosekunden ist der Verlauf der magnetischen Feldstärke in $10^{-3}$ A/m aufgetragen.

Fig. 1(b) zeigt das zugehörige normierte Frequenzspektrum im Bereich von 0 bis 100 kHz. Es ist ersichtlich, daß der überwiegende Frequenzanteil im Bereich von 10 kHz liegt. Es ist dies eine typische Impulsstrahlung nB. Gleichzeitig zeigt jedoch die Frequenzanalyse nach Fig. 1(b), daß noch weitere Frequenz-Anteile enthalten sind. Diese zusätzlichen Anteile werden also mittels der beschriebenen Vorrichtung ebenfalls erfaßt, d. h. aufgenommen und gespeichert. Zur Durchführung des erfindungsgemäßen Verfahrens wird die 10 kHz-Impulsstrahlung nB der in Fig. 1(a) und 1(b) gezeigten Art reproduziert.

Die erforderliche Wiedergabevorrichtung zeigt Fig. 2(b). Die zuvor im Rechner 7 auf z.B. einer Diskette abgespeicherten Signalverläufe werden von einem Rechner 11, in dem dieser vorerwähnte transportable Speicher 10 eingebracht oder in dessen Speicher zuvor die auf dem Speicher 10 abgespeicherten Impulsstrahlung übertragen werden, über den XT-Bus 12 auf einen programmierbaren Signalgenerator 20 übertragen. Der Rechner 11 kann im Prinzip ein Rechner wie der Rechner 7 sein. Aufnahme und Wiedergabe können jedoch zu verschiedenen Zeitpunkten und an verschiedenen Orten stattfinden. Der Rechner 11 veranlaßt den Signal-Generator 20 zufallsgesteuert in zeitlichen Abständen, die zwischen 50 und 150 Millisekunden variieren, den Signalverlauf der in Speicher 10 gespeicherten Impulsstrahlung auszulesen. Der magnetische Feldverlauf des Absolutbetrages der Impulsstrahlung wird mit einer Helmzholtz-Spule 14 simuliert. Da hierbei der Strom durch die Helmzholtz-Spule 14 die maßgebliche Größe ist (das erzeugte Magnetfeld ist diesem Strom proportional), muß dieser vorher mit Hilfe des Spannungs/Stromwandlers 13 auf das Ausgangssignal des Signalgenerators 20 eingeprägt werden. Damit wird innerhalb der Helmzholtz-Spule 14 ein im wesentlichen homogenes Magnetfeld erzeugt, das in Verlauf und Frequenzcharakteristik gleich dem der aufgenommenen natürlichen Impulsstrahlung ist. Damit wird diese Impulsstrahlung also reproduziert. Es entsteht also eine künstliche ("artifizielle") Impulsstrahlung.

Die Helmholtz-Spule 14 hat einen Durchmesser von circa 20 cm und einen Abstand der Teilspulen von 10 cm. Diese Angaben gelten für die Anordnung, mit der die C6-Glioma-Zellen (siehe weiter unten, Ziff. 1) untersucht wurden.

## 1. C6-Glioma-Zellen

Zur Untersuchung der Auswirkung des in dieser Art erzeugten -in Fig. 2(b) durch die Pfeile angedeuteten - magnetischen Feldes der artifiziellen Impulsstrahlung wurden C6-Glioma-Zellen als Probe 16 in dem Raum zwischen den Spulen angeordnet. Die gesamte Anordnung erfolgte in einem Inkubator 15. Dabei ist darauf zu achten, daß die Probe 16 in der Mittelachse der Helmzholtz-Spule 14 angeordnet wird, da dort das magnetische Feld am homogensten ist. Die Inhomogenität der magnetische Feldstärke innerhalb der Helmholtz-Spule betrug bei einer praktischen Realisierung 40 bis 200% des Sollwertes. Die nominelle magnetische Maximal-Feldstärke betrug 100 mA/m bzw. 25 mA/m. Im Vergleich dazu beträgt die durchschnittliche magnetische Feldstärke einer natürlichen Impulsstrahlung circa 8 mA/m.

Die Zellkultur von C6-Glioma-Zellen wurde wie folgt gewonnen: Die C6-Glioma-Zellen wuchsen als Monolayer in Petrischalen (100.20 mm, Falcon 3003, Becton Dickinson, Plymouth, England) auf Dulbecco Modified Minimum Essential Medium (DMEM; Boehringer Mannheim, Deutschland) mit 25 mM Hydrogencarbonat. Das Medium wurde mit 10% fötalem Kälberserum (FCS; Boehringer Mannheim) ergänzt. 100 IU/ml Penicillin G 50 und 50 $\mu$g/ml Streptomycin wurden zur Verhinderung bakterieller Infektionen hinzugeben. Die Zellen wurden bei 37 °C in angefeuchteter Raumluft mit 5% CO2 in einem herkömmlichen Inkubator kultiviert. Drei Mal wöchentlich wurden die Zellen passagiert. Für jedes Experiment wurden acht (8) vollständig mit Zellen bedeckte Petrischalen, die zwei Tage nach dem Passagieren erhalten wurden, eingesetzt. Die Zellen wurden geerntet durch kurzzeitige Inkubation mit 0,06% Trypsin/0,02% EDTA, in PBS (phosphate buffered saline) ausgesetzt, in FCS-haltigem Medium resuspendiert, gepoolt und danach in gleichen Mengen in 24 Petrischalen (60.25 mm, Falcon 3004, Becton Dickinson) ausgesät.

Zur Durchführung des Versuchs wurden 2 Gruppen A und B von je 12 Petrischalen je in die Helmholtz-Spule 14 und eine weitere Helmholtz-Spule 17 in einem Inkubator 5 als Proben 16 und 18 eingesetzt. Beide Gruppen wurden soweit voneinander entfernt wie möglich plaziert. Die Helmholtz-Spule 14 mit Gruppe B wurde in der bereits beschriebenen Weise elektromagnetisch erregt; die andere Helmholtz-Spule 17 mit Gruppe A als Probe 18 wurde kurzgeschlossen und diente als Kontrolle. Nach einer Inkubationszeit von 24 Stunden wurde das Medium entfernt und die Kulturen mit PBS ausgewaschen. Dann wurde in jede Schale 5 ml DMEM mit 10% FCS und $^3$H-Thymidin (3,7.10$^4$ Bq/ml; Amersham Buchler, Braunschweig, Deutschland) gegeben. Nach einer weiteren Stunde Inkubation der Kulturen innerhalb der Spule wurde das Medium sorgfältig entfernt. Die Zellen wurden mit eiskaltem PBS gewaschen und die Schalen auf Eis gestellt. Danach wurden die Zellen jeder Petrischale separat in der oben beschriebenen Art geerntet und die Zellen bis zur Bestimmung des DNA-Gehaltes und der $^3$H-Thymidin-Inkorporation eingefroren. In jeder Probe wurde die spezifische Radioaktivität in cpm/$\mu$g DNA bestimmt und für die beiden Gruppen A und B der Mittelwert gebildet. Das Ergebnis für Gruppe B wurde prozentual auf das der Gruppe A (Kontrolle) bezogen.

Bei den Experimenten wurden drei Versuchsreihen durchgeführt. In einer Kontrollreihe mit 10 Versuchen wurde keine der Gruppen A und B der artifiziellen Impulsstrahlung ausgesetzt. In zwei Versuchsreihen wurde die Gruppe B einer Impulsstrahlung mit einem überwiegenden Spektralanteil von 10 kHz bei magnetischen Maximal-Feldstärken von 100 mA/m (13 Versuche) und 25 mA/m (4 Versuche) ausgesetzt.

Der erste Schritt zur Bestimmung der spezifischen Radioaktivität war die Extraktion der pulsmarkierten DNA aus den C6-Glioma-Zellen nach Weinbren und Woodward **(Brit.J.exper.Path.45, 442-449, 1964)**.

Nach Auftauen der gefrorenen Zellproben (suspendiert in je 1 ml PBS) wurde 3 ml gekühlte 0,25 M Perchloressigsäure (PCA) zugegeben. Die Proben wurden dann 30 Minuten lang bei 4 °C stehen gelassen. Die Zellen wurden dann 15 Minuten bei 4.000xg zentrifugiert und die Pellets in 0,5 ml 0,5M NaOH bei Raumtemperatur 30 Minuten lang geschüttelt. Danach wurden 4,5 ml 0,5 M PCA hinzugegeben und die Proben wieder bei 4 °C zumindest 30 Minuten lang (oder über Nacht) stehengelassen. Die Proberöhrchen wurden 15 Minuten lang bei 4.000xg zentrifugiert und die Pellets bei 95 °C in 3 ml 0,5M PCA jeweils 20 Minuten lang gekocht. Nach 10minütiger Zentrifugierung bei 4.000xg wurden die Überstände gewonnen und aufbewahrt. Dieses Verfahren wurde wiederholt, die beiden Überstände vereinigt und ihr ($^3$H)-Thymidin-Gehalt sowie ihr DNA-Gehalt nach Burton **(BURTON, K., Biochem. 62 (1956), pp. 315 - 323)** bestimmt. Zu 0,5 ml des Überstandes wurde 1 ml einer Lösung von Diphenylamin in Essigsäure zugegegeben. Die Proben wurden bei Raumtemperatur im Dunkeln 22 bis 24 Stunden aufbewahrt. Dann wurde die Extinktion bei 590 nm gemessen und der DNA-Gehalt berechnet.

Die spezifische Radioaktivität ergab sich dann als Quotient der Werte der Radioaktivität und der DNA, ausgedrückt in dpm/µg DNA.

Aus den Kontrollergebnissen ohne Bestrahlung ergab sich, daß die Ergebnisse sich je nach Anordung im Inkubator um 3.9 ± 1,4% (Mittelwert ± Standardabweichung) hinsichtlich der Inkorporation von ($^3$H)-Thymidin in die nukleare DNA unterschieden. D. h., es gab - auch ohne Bestrahlung - eine bestimmte Stelle im Inkubator (nämlich an der Grundfläche desselben), an der sich eine höhere Proliferationsrate der Zellen als an anderer Stelle ergab. Dieser Wert (3,9 ± 1,4%) wurde daher anschließend von den Meßergebnissen bei Bestrahlung abgezogen, um evtl. Beeinflussungen durch magnetische Felder im Inkubator rechnerisch auszugleichen.

Für die Gruppe B (Bestrahlung mit artifiziellen Impulsstrahlung mit überwiegendem Spektralanteil von 10 kHz) ergab sich bei einer Feldstärke von 100 mA/m eine Proliferationsrate von -4.5 ± 3,7% (p __ 0.05), bei einer magnetischen Feldstärke von 25 mA/m eine Proliferationsrate von 6.1 ± 1.8%, bezogen auf A. Korrigiert man diese Werte um den oben angegebenen Wert, der zur Kompensation von Einflüssen des Inkubators berücksichtigt werden muß, so ergeben sich folgende Werte:

| Feldstärke | Proliferationsrate |
|---|---|
| 0 | 0 |
| 25 mA/m | 2,2 ± 1,8% |
| 100 mA/m | -8,4 ± 3,7% |

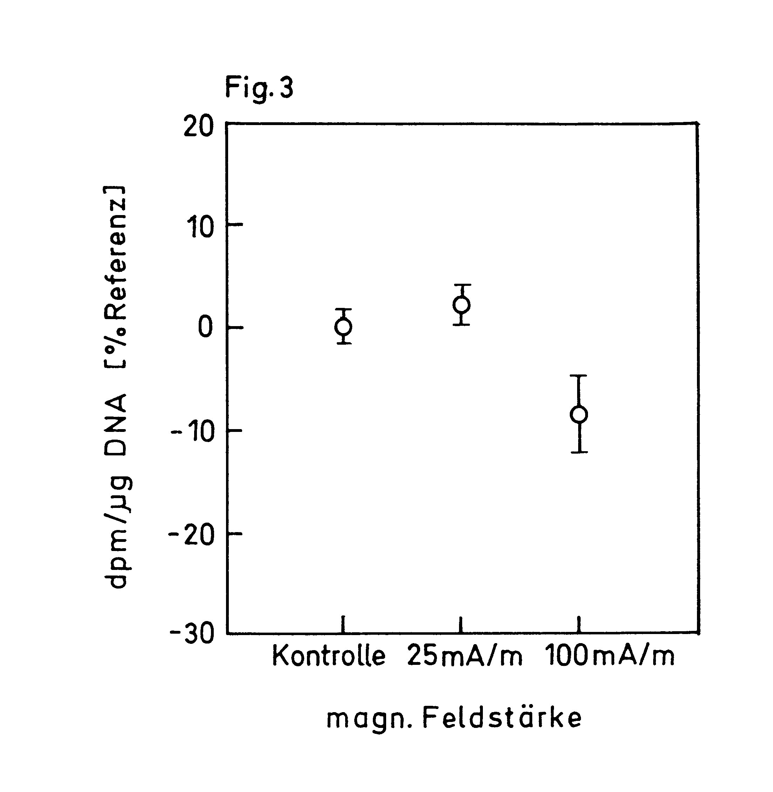

Das Ergebnis ist in Fig. 3 dargestellt.

Es ergibt sich also, daß bei geringer Feldstärke (25 mA/m) zunächst ein kleiner Anstieg der Proliferationsrate erfolgt, während bei höherer Feldstärke (100 mA/m) eine signifikante Verringerung der Proliferationsrate der DNA erfolgt.

Das Ergebnis deutet daraufhin, daß bestimmte Spektren, die durch breitbandigen Empfang natürlicher Impulsstrahlung erhalten werden, die Proliferationsaktivität der C6-Glioma-Zellen beeinflussen. Ihre meßbare Wirkung bei künstlicher Erzeugung der artifiziellen Impulsstrahlung deutet darauf hin, daß in der natürlichen Impulsstrahlung Strahlungsanteile enthalten sind, die nicht nur Indikatoren biologischer Wirkungen, sondern ihre Ursache sind.

## 2. Ascites-Hepatom-Ratten

Ein weiteres Beispiel der Beeinflussung von Eigenschaften biologischen Materials ist die Bestrahlung von Ratten, die mit einem Ascites Hepatom infiziert sind.

Als Testobjekt wurden Yoshida-Tumor-Ratten verwendet. Bei ihnen wurde durch Injektion der Yoshida Zellen ein Yoshida-Ascites-Ratten-Hepatom AH130 erzeugt. Nach intraperitonealer (i. p.) Injektion entwickelte sich das Hepatom in 11 bis 14 Tagen.

Mit artifizieller Impulsstrahlung, deren Erzeugung oben beschrieben worden ist, und zwar mit einem überwiegenden Anteil von 10 kHz, wurden 10 bzw. 20 Ascites-Ratten bestrahlt. Sie wurden mit 10 nicht bestrahlten Tieren (Kontrolle) verglichen.

Dieser Vergleich wurde sechs Mal mit künstlicher Impulsstrahlung mit spektralem Schwerpunkt von 10 kHz mit einer maximalen Feldstärke von 25 mA/m durchgeführt. In allen sechs Fällen lebten die bestrahlten Gruppen im Mittel länger als die Kontrollen, und zwar im Durchschnitt vom ersten Todestag an etwa um 30%.

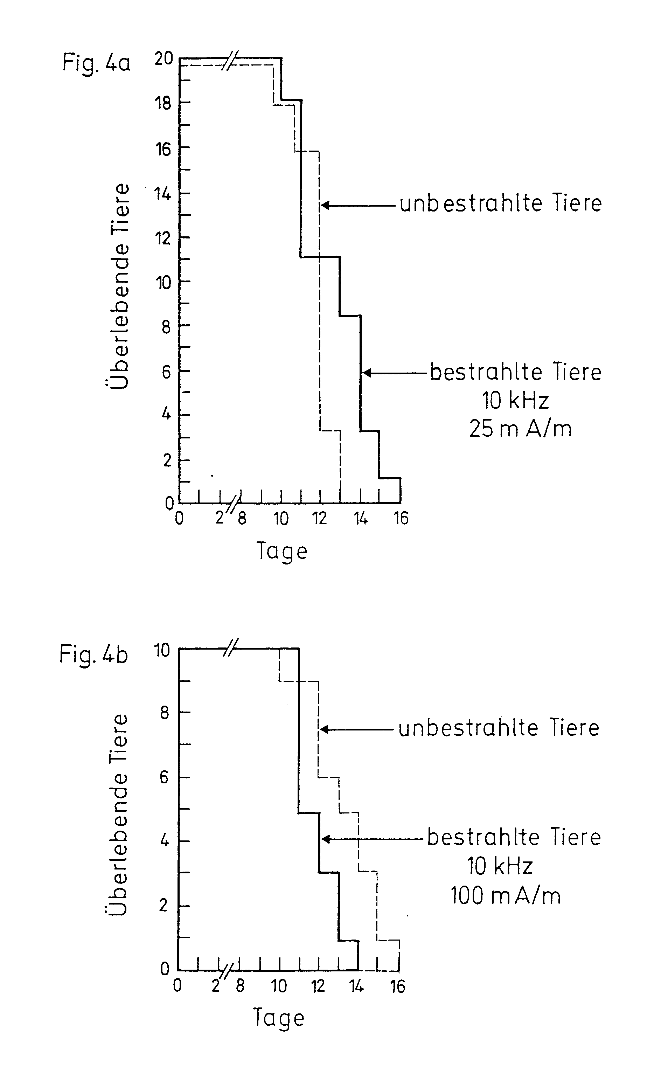

Dieser Vergleich wurde ferner drei Mal mit einer maximalen Feldstärke von 100 mA/m durchgeführt. In diesem Fall lebten alle drei Male die bestrahlten Tiere um durchschnittlich etwa 30% kürzer als die Kontrolle. Die Ergebnisse sind in Abbildungen 4(a) und 4(b) dargestellt.

Fig. 4(a) zeigt die Überlebenszeit von 20 Yoshida-Ascites-Hepatom-Ratten nach einer Bestrahlung mit 10 kHz Impulsstrahlung bei einer Feldstärke von 25 mA/m. Fig. 4(b) zeigt das Ergebnis bei mit einer Feldstärke von 100 mA/m. Die Ergebnisse für die nicht bestrahlte Kontrolle sind in strichpunktierten Linien, die Ergebnisse für die bestrahlten Versuchstiere in durchgezogenen Linien dargestellt.

Es ergibt sich also, daß die Bestrahlung mit der geringeren Intensität (25 mA/m) die Überlebenszeit der infizierten Ratten verlängert.

## 3. Verschiebung des EEG

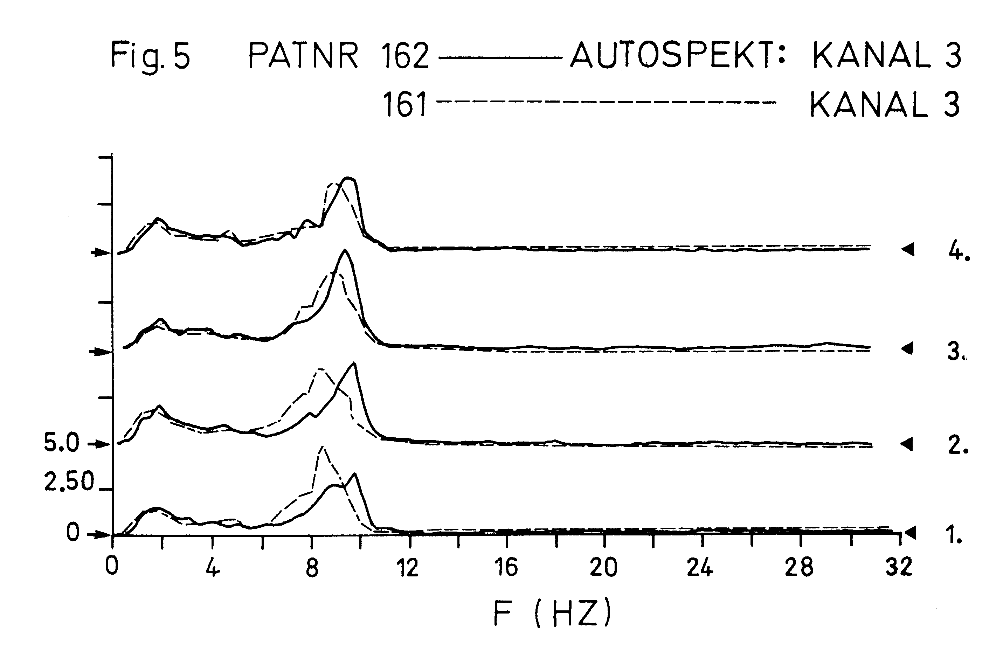

Ein Bestrahlungsgerät aus zwei Helmholtz-Spulen wurde ferner so ausgestaltet, daß man bei menschlichen Probanden eine Ganzkörperbestrahlung durchführen konnte. Hierzu wurde bei einem Bett die eine Helmholtz-Spule in die Höhe der Matratze gelegt; die andere wurde ca. 1 m höher angeordnet. Auf das Bett wurden die Probanden gelegt und damit dem einigermaßen gleichartigen magnetischen Feld künstlicher Impulsstrahlung ausgesetzt. Es wurden etwa 20 gesunde Jugendliche auf diese Weise bestrahlt und dabei deutliche Verschiebungen im EEG festgestellt. Ein typisches Ergebnis ist in Fig. 5 dargestellt. Es zeigt in gestrichelten Linien das Frequenzspektrum EEG ohne Beeinflussung durch Impulsbestrahlung, in durchgezogenen Linien das EEG unter Beeinflussung durch künstliche Impulsstrahlung bei einer magnetischen Feldstärke von 25 mA/m und einem überwiegendem Frequenzanteil von 8 kHz.

Im einzelnen zeigt Fig. 5 Ausdrucke von Autospektren, gewonnen aus der okzipitalen EEG-Ableitung des Probanden. Die gestrichelte Linie stellen einen 4-Minuten-Abschnitt aus einer 10-Minuten-Ableitung bei einem Patienten dar, unterteilt in 4 konsekutive übereinander (1., 2., 3., 4.) dargestellte 4-Minuten-Abschnitte etwa aus der Mitte der 10-Minuten-Epoche. Die durchgezogenen Linien stellen das Autospektrum desselben Probanden unter Magnetfeldbestrahlung mit 8 kHz und 25 mA/m dar. Gegenüber der Null-Ableitung ist eine deutliche Verschiebung in dem schnelleren Anteil des dominierenden Gipfels zu ersehen. Die Gipfel in den sehr langsamen Frequenzen sind vorwiegend Artefacte. Bemerkenswert ist die fehlende Verschiebung bei 22 Hz.

Die Hauptänderung nach Bestrahlung spielt sich also im Bereich der sogenannten Grundaktivität ab, die bei beiden Probanden im $\alpha$-Bereich (7,5-13,5 Hz) liegt. (Zur Meßmethode vgl. G. Ruhenstroth-Bauer, Intern. J. Neuroscience, im Druck).

Auch dieser Befund belegt - sogar beim Menschen - die Wirkung der mit Hilfe des erfindungsgemäßen Verfahrens und Gerätes gezielt beherrschbar gemachten Impulsstrahlung.

## Patentansprüche

1. Verfahren zur Bereitstellung eines Gerätes zur Beeinflussung derjenigen Eigenschaften biologischen Materials, die mit dem Auftreten natürlicher Impulsstrahlung korrelieren, **gekennzeichnet durch** folgende Schritte:
   (a) Empfang und Speicherung des zeitlichen Verlaufes des magnetischen Feldes natürlicher Impulsstrahlung;
   (b) Erzeugung artifizieller Impulsstrahlung durch Reproduktion des gespeicherten magnetischen Feldes in einer Spulenanordnung (16);
   (c) Einbringung des biologischen Materials in das nach (b) erzeugte magnetische Feld.

2. Verfahren nach Anspruch 1, dadurch gekennzeichnet, daß die zur Aufnahme und Speicherung bestimmten Impulse der natürlichen Impulsstrahlung einen ausgeprägten Frequenzanteil bei 8 oder 10 kHz haben.

3. Gerät zur Beeinflussung biologischer und pathologischer Parameter biologischen Materials, gekennzeichnet durch
   (a) einen Speicher (10) zur Speicherung von Signalen, die den zeitlichen Verlauf von Teilen natürlicher Impulsstrahlung darstellen;
   (b) Mittel (11,12,13,20) zum zufallsgesteuerten Auslesen der im Speicher (10) gespeicherten Signale;
   (c) einen Magnetfeld-Generator (14), dessen Feldstärke in einem bestimmten, räumlichen Bereich weitgehend homogen ist, und der mit den aus dem Speicher (10) ausgelesenen Signalen als artifizielle Impulsstrahlung ein Magnetfeld erzeugt;
   (d) Mittel (16) im Bereich des homogenen Feldes zum Einbringen biologischen Materials.

4. Gerät nach Anspruch 3, gekennzeichnet durch seine Verwendung zur Behandlung von mit einem Ascites-Hepatom infizierten biologischen Material.

5. Gerät nach Anspruch 3, gekennzeichnet durch seine Verwendung zur Verminderung der Proliferationsrate von Tumorzellen.

6. Gerät nach Anspruch 3, gekennzeichnet durch die Verwendung von Impulsstrahlung mit einem ausgeprägten Frequenzanteil von 10 kHz.

7. Gerät nach Anspruch 3, gekennzeichnet dadurch, daß der Magnetfeld-Generator durch Helmholtz-Spulen (14,17) gebildet wird.

8. Gerät nach Anspruch 3, gekennzeichnet durch Anordnung von zwei Spulen des Magnetfeldgenerators oberhalb und unterhalb einer zum Liegen eines Probanden geeigneten Fläche.

rel. Einheit

mA/m

−5.5

5.5

0

Fig. 1a

Fig. 1b

0

1

0

400

800 µs

0

50

100 kHz

Fig. 2a

Fig. 2b

EP 0 642 806 A2

Fig. 3

10

Fig. 4a

Überlebende Tiere / Tage

unbestrahlte Tiere

bestrahlte Tiere
10 kHz
25 m A/m

Fig. 4b

Überlebende Tiere / Tage

unbestrahlte Tiere

bestrahlte Tiere
10 kHz
100 m A/m

11

Fig. 5    PATNR 162 ————————— AUTOSPEKT: KANAL 3
              161 —————————————— KANAL 3

F (HZ)